Europäisches Patentamt

European Patent Office

Office européen des brevets

(19)

(11) Publication number: **0 315 950**
**A2**

## (12) EUROPEAN PATENT APPLICATION

(21) Application number: 88118576.3

(51) Int. Cl.⁴: **C12N 15/00 , C12N 1/38**

(22) Date of filing: 08.11.88

The applicant has filed a statement in accordance with Rule 28 (4) EPC (issue of a sample only to an expert). Accession number(s) of the deposit(s): FERM BP-949, FERM BP-2089, FERM BP-1296.

(30) Priority: 09.11.87 JP 283777/87

(43) Date of publication of application:
**17.05.89 Bulletin 89/20**

(84) Designated Contracting States:
**CH DE ES FR GB IT LI NL SE**

(71) Applicant: **OTSUKA PHARMACEUTICAL CO., LTD.**
**9, Kandatsukasacho 2-chome**
**Chiyoda-ku Tokyo 101(JP)**

(72) Inventor: **Takano, Masaaki**
**77-6, Aza-Inui Sumiyoshi Aizumi-cho**
**Itano-gun Tokushima-ken(JP)**
Inventor: **Kamogashira, Takashi**
**68-8, Nakayoshino-cho 1-chome**
**Tokushima-shi Tokushima-ken(JP)**
Inventor: **Hirai, Yoshikatsu**
**5-49, Aza-Ekogawa Shinkirai Kitajima-cho**
**Itano-gun Tokushima-ken(JP)**

(74) Representative: **Vossius & Partner**
**Siebertstrasse 4 P.O. Box 86 07 67**
**D-8000 München 86(DE)**

(54) Method of incubating cells expressing a recombinant protein.

(57) Disclosed is a method of incubating cells expressing a recombinant protein or polypeptide wherein the expressed protein or polypeptide is obtained withot the N-terminal Met-residue encoded by the translation initiation codon which comprises incubating in a culture medium a host cell transformed wth a cytoplasmic expression vector containing the gene of a protein or polypeptide wherein the amount of oxygen dissolved in the culture medium is at most about 20% of the saturation concentration.

EP 0 315 950 A2

# METHOD OF INCUBATING CELLS EXPRESSING A RECOMBINANT PROTEIN

The present invention relates to a novel method of incubating cells expressing a recombinant protein. More particularly it relates to a novel method of incubating such cells wherein the amount of dissolved oxygen in the culture medium is controlled. According to this method a useful polypeptide is obtained which does not contain a methionine residue (hereinafter referred to as "Met") which corresponds to a translation initiation codon at its N-terminus.

Remarkable advances have been made in recent years in the field of biochemistry, especially in gene recombination techniques. Various gene engineering techniques have been developed for preparing large quantities of physiologically active substances, such as lymphokines, which were obtained only in small amounts in the past.

These techniques include a method of producing the desired useful polypeptide in a large quantity by introducing the gene encoding the polypeptide into a vector of a microorganism such as E. coli and causing the transformed cells of the microorganism to express the polypeptide by replication, transcription and translation. This method is known as the most typical and advantageous method.

In preparing useful polypeptides by such gene engineering techniques, the translation of the polypeptide is initiated at the initiation codon (ATG) encoding a methionine residue regardless of whether the host cell is a eucaryotic or a procaryotic cell, so that the amino terminal (N-terminal) amino acid of the polypeptide obtained is Met. It is known that in the case of polypeptides naturally produced by cells of organisms, the N-terminal Met is cut off with enzymes such as aminopeptidases in the host cell (J.D. Watson, Molecular Biology of the Gene, (I), 3rd Edition, translated by Kin-ichiro Miura et al., p. 375 (1977)).

However, in the case of cells transformed by a vector having introduced therein a gene for a peptide of foreign origin, such as interferon-α (IFN-α), growth hormone (GH) or interleukin-2 (IL-2), especially in the case where a large quantity of useful polypeptide is sought to be prepared by incubating such cells, the enzyme present in the host cell is unable to fully exhibit its activity, permitting the production of large amounts of polypeptides retaining their N-terminal Met residue (see Nature, 293, 408 (1981), etc.). It is known that polypeptides (e.g. IL-1α and IL-1β) with an N-terminal Met residue display a lower biological activity than natural polypeptides free of the N-terminal Met residue. Furthermore, polypeptides, which are not occurring naturally, might possibly have new antigenic properties. The use of the polypeptide as a drug is therefore likely to involve various restrictions and shortcomings.

Recently, it has been proposed to cause enzymes such as a methionine aminopeptidase [A. BEN-BASSAT et al., J. Bact., 169, 751-757(1987); C.G. Miller et al., P.N.A.S., 84, 2718(1987)] or the aminopeptidase M [S. Nakagawa et al., Bio/Technol., 5, 824(1987)] to act on polypeptides having the N-terminal Met residue in order to remove the Met. Nevertheless, this method is only useful for the polypeptides having a specific sequence but is not for a wide variety of other polypeptides. Additionally, the enzyme used is expensive. The method therefore still remains to be improved for actual commercial use.

Thus it is an object of the invention to provide an easy method of removing the N-terminal Met residue of a given polypeptide which is useful for a wide variety of desired polypeptides regardless of their particular origin or amino acid sequence and which is suitable for large scale industrial operations and also is economically satisfactory.

The present invention provides a method of incubating cells expressing a recombinant protein or polypeptide wherein the expressed protein or polypeptide is obtained without the N-terminal Met-residue encoded by the translation initiation codon which comprises incubating in a culture medium a host cell transformed with a cytoplasmic expressin vector containing the gene of a protein or polypeptide wherein the amount of oxygen dissolved in the culture medium is at most about 20% of the saturation concentration.

According to the present invention it was found that when cells expressing a recombinant protein are incubated in a culture medium, the proportion of the desired polypeptide free of the N-terminal Met residue is dependent on the amount of the oxygen dissolved in the medium, and that the desired Met-free polypeptide can be obtained substantially selectively by suitably controlling the amount of dissolved oxygen. The present invention has been accomplished based on these findings.

According to the present invention, the desired polypeptide free of the N-terminal Met residue can be obtained substantially selectively regardless of the kind of polypeptide by the surprisingly simple procedure of incubating a host cell transformed with a cytoplasmic expression vector containing a gene encoding a protein or polypeptide in a suitable culture medium containing a particular amount of dissolved oxygen. The method of the invention is therefore suitable for industrial operations and can be practiced economically advantageous without using any expensive enzyme.

The method of the present invention is suitable for the production of any desired polypeptide, in particular of those having an amino acid sequence already known or encoded by a known DNA sequence. Examples of such polypeptides are interleukin-1 (IL-1), interleukin-2 (IL-2), interleukin-3 (IL-3), interleukin-4 (IL-4), interleukin-5 (IL-5), interleukin-6 (IL-6), colony stimulating factors (M-CSF, GM-CSF, G-CSF, etc.), tumor necrosis factor (TNF), lymphotoxin (LT) and other lymphokines.

Useful host cells are those which are generally known for use in cytoplasmic expression systems, i.e. in the protein expression system wherein a vector is used which contains the gene of the desired polypeptide linked to and immediately following a translation initiation codon. Examples of such host cells are E. coli, Bacillus subtilis, Diplococcus pneumoniae, yeast or actinomycetes.

These host cells can be transformed by known methods. The expression vectors for use in preparing the transformant, the gene coding for the desired polypeptide constituting the vector, various regulators for expressing the gene, such as a promoter, a sequence coding for a ribosome binding site and a transcription terminator, and vectors as a starting material for incorporating said genes are also known. Alternatively, they can be easily prepared by known methods or procedures which are generally used in gene recombination techniques.

The method of the invention is especially advantageous for expressing recombinant IL-1$\alpha$ and IL-1$\beta$ and derivatives thereof in E. coli.

While the present invention will be described below in greater detail with reference to a system for the cytoplasmic expression of IL-1 by E. coli, other cases can be handled in substantially the same way.

The IL-1$\alpha$ gene or IL-1$\beta$ gene to be used in the present method can be totally synthesized according to the amino acid sequence of the known natural IL-1$\alpha$ or IL-1$\beta$ by known methods, for example by the phosphite triester method (Nature, 310, 105(1984)) or by other methods for the chemical synthesis of nucleic acids, or it can be extracted and isolated by conventional methods from sources such as cells containing the gene. Furthermore, such IL-1 genes were already isolated. Examples of them are those disclosed in the following literature :

* Nishida et al., B.B.R.C., 143, 345(1987)
* Frutani et al., Nucleic Acid Res., 13, 5869(1985)
* European Patent Application published under publication number 237967
* European Patent Application published under publication number 237073

The promoter to be used for expressing the gene can be any of various known ones, such as the $\lambda P_L$, $\lambda P_R$, trp, lacC, tufB, recA or 1pp promoter, among which the trp promoter is particularly preferred and advantageous.

The cytoplasmic expression vectors containing the IL-1 gene and such a promoter which are particularly useful in the method of the present invention may in addition to those given in the above literature also contain those disclosed for example in Kikumoto et al., B.B.R.C., 147, 315(1987).

These plasmids are introduced by conventional methods into E. coli as host cells whereby the E. coli is transformed.

The culture medium to be used for incubating the resulting transformant can be any of various synthetic or natural media which are conventionally used for growing E. coli and producing the contemplated polypeptide. The carbon sources usually used include glucose, fructose, lactose, glycerol, mannitol or sorbitol. Examples of useful nitrogen sources are ammonium chloride (NH$_4$Cl), ammonium sulfate [(NH$_4$)$_2$SO$_4$], Casamino acid, yeast extract, polypeptone, meat extract, Bacto-trypton or corn steep liquor. Examples of other useful nutrients are K$_2$HPO$_4$, KH$_2$PO$_4$, NaCl, MgSO$_4$, vitamin B1, or MgCl$_2$. For example the medium of the following composition is especially suitable for incubating an E. coli transformed with an expression vector containing the trp promoter:

| Na$_2$HPO$_4$·12H$_2$O | 6 g/liter |
|---|---|
| KH$_2$PO$_4$ | 3 g/liter |
| NaCl | 0.5 g/liter |
| NH$_4$Cl | 1 g/liter |
| Bacto-Casamino acid | 10 g/liter |
| Bacto-Yeast extract | 0.5 g/liter |
| L-Cysteine HCl | 75 mg/liter |
| L-Proline | 75 mg/liter |
| L-Leucine | 75 mg/liter |

The above medium is adjusted to pH 7.4 (with 4N NaOH), and is thereafter autoclaved at 121°C for 30 minutes or heated with steam at 123°C for 20 minutes for sterilization. Subsequently, the following mixture which is sterilized separately is aseptically added to the medium immediately before inoculation:

| 1M MgSO$_4$·7H$_2$O | 2 ml/liter |
| 1M CaCl$_2$·2H$_2$O | 0.1 ml/liter |
| 7.5 mg/ml Thiamine HCl | 1 ml/liter |
| 40% Glucose | 18.75 ml/liter |

Preferably, the transformant is incubated with aeration and stirring under conditions suitable for the growth of the transformant, usually at a pH 5.5 to 8.5 at a temperature of 18 to 40°C.

According to the invention, the amount of oxygen dissolved in the medium is critical in the above incubation and may not exceed 20% of the saturation concentration. Preferably it is limited to about 0 to 15%, more preferably to about 0 to about 5%. The concentration of dissolved oxygen is limited in the method of the present invention when the transformants start to express the protein during the incubation, i.e. in the logarithmic growth phase, particularly in the final stage of the logarithmic growth phase. The concentration of dissolved oxygen can be controlled, for example, by suitably varying the aeration condition or stirring conditions. The details thereof will be described in the examples to follow.

According to the method of the present invention, the desired useful polypeptide can be obtained without the Met residue which corresponds to the initiation codon of the expressed protein.

The production of the desired polypeptide can be detected by usual methods, such as an enzyme immunoassay (EIA).

The desired polypeptide can be isolated and purified by extraction from the resulting cells according to conventional methods, such as sonication or the method of Laemmeli using SDS-buffer (Laemmeli, U.K., Nature, 227, 680(1970)).

The method of the present invention provides substantially selectively the desired useful polypeptide without the N-terminal Met residue. Nevertheless, the product is likely to contain a polypeptide with Met. The product can be checked for the presence of such polypeptides with met for example by subjecting the product to gel chromatography using TSK-SP-5PW (column 7.5 x 75 mm, product of Toyo Soda Mfg. Co., Ltd.) and measuring the absorbance of the fractions at 280 nm, or by measuring the N-terminal Met residue content by dansylation or with a peptide sequencer (applied Biosystems).

The desired polypeptide prepared by the method of the present invention can be purified by various conventional procedure utilizing the physical or chemical properties of the desired polypeptide (see for example, "Biological Data Book II," pp. 1175-1259, 1st edition, 1st print, June 23, 1980, published by Kabushiki Kaisha Tokyo Kagakudojin). It is desirable to extract the polypeptide from the host under a mild condition such as by osmotic shock in order to maintain the higher-order structure thereof. Examples of useful procedures for purification are the treatment using a conventional protein precipitating agent, ultrafiltration, molecular sieve chromatography (gel filtration), liquid chromatography, centrifugation, electrophoresis, affinity chromatography, or dialysis, or combinations of such procedures.

Specifically, the above procedure can be carried out, for example, as follows. First, the desired polypeptide is partially purified from a supernatant obtained upon extraction of the cells. This partial purification is carried out, for example, by a treatment using as a protein precipitating agent an organic solvent such as acetone, methanol, ethanol, propanol or dimethylformamide (DMF), or an acid such as acetic acid, perchloric acid (PCA) or trichloroacetic acid (TCA), a treatment using a salting-out agent such as ammonium sulfate, sodium sulfate or sodium phosphate and/or ultrafiltration using a dialysis membrane, flat membrane or a hollow fiber membrane. These treatments are conducted in the same manner as usually done under usual conditions.

The roughly purified product thus obtained is then subjected to gel filtration, whereby a fraction exhibiting the activity of the desired polypeptide is collected. Useful gel filtration agents are not limited specifically. Such agents include those made of dextran gel, polyacrylamide gel, agarose gel, polyacrylamide-agarose gel or cellulose. Examples of useful gel filtration agents are those commercially available such as Sephadex G type, Sephadex LH type, Sepharose type, Sephacryl type (all products of Pharmacia), Cellofine (Chisso Corporation), Biogel P type, Biogel A type (both product of Bio-Rad Lab), Ultro gel-C (product of LKB) or TSK-G type (product of Toyo Soda Mfg. Co., Ltd.).

The desired polypeptide can be isolated as a homogeneous substance from the fraction exhibiting the activity of the desired polypeptide. for example by subjecting the fraction to affinity chromatography using a hydroxyapatite column, to ion exchange column chromatograpy as of the DEAE, CM or SP method, to a chromatofocusing method or to a reverse-phase high-performance liquid chromatography, or to a combina-

4

tion of such methods.

The chromatofocusing method can be carried out by various known procedures. Useful as a column is for example PBE94 (Pharmacia Fine Chemicals), as the starting buffer for example imidazole-hydrochloric acid, and as the eluent for example a mixture of Polybuffer 74 (Pharmacia Fine Chemicals) and hydrochloric acid (pH 4.0).

The reverse-phase high-performance liquid chromatography can be conducted for example with $C_4$ Hi-Pore reverse-phase HPLC column (Bio-Rad Laboratories) using acetonitrile, trifluoroacetic acid (TFA) or water, or a mixture of such solvents as the eluent. In this way, the polypeptide can be obtained upon isolation without the N-terminal Met residue.

The desired polypeptide thus obtained has the same physiological activities as the corresponding naturally occuring polypeptide and is equally useful for preparing medicinal preparations which are the same as those containing the naturally occurring polypeptide.

These medicinal preparations are usually formulated in the form of pharmaceutical compositions comprising an effective amount of the polypeptide and a suitable pharmaceutical carrier. Examples of useful pharmaceutical carriers include excipients and diluents such as filler, extender, binder, wetting agent, disintegrator or surfactant which are generally used for preparing pharmaceuticals of the desired form to be used. The form of the pharmaceutical compositions is not specifically limited insofar as they effectively contain the polypeptide prepared according to the invention and can be, for example, in the form of tablets power, granules, pellets or as solid preparation. Usually, however, it is suitable that the composition be in the form of a solution, suspension or emulsion for injection. Alternatively, such a composition can be a dry product which can be made liquid with addition of a suitable carrier before use. The pharmaceutical compositions mentioned above can be preapred by usual methods.

In accordance with the form of the pharmaceutical composition obtained, the composition is administered via a suitable route. For example those for injection are given intravenously, intramuscularly, subcutaneously, intracutaneously or intraperitoneally. The solid composition is given orally or intraintestinally. The amount of the active component of the composition and the dosage of the composition are suitably determined according to the method and form of administration, purpose of use, symptoms of the patient, etc. and are not definitely determinable. It is generally desirable to incorporate about 1 to about 80 wt. % of the active component into the pharmaceutical preparation and to give the preparation at a daily dose of about 0.1 $\mu$g to about 10 mg, calculated as the active component, for human adults. The preparation need not always be given only once a day but can be given in three to four divided doses daily.

The present invention will be described in greater detail with reference to the following examples.

In the following examples, physiological activities were determined by the following methods.

### (1) Determination of IL-1$\alpha$ activity

Expressed in terms of LAF activity as measured by the method of J. J. Oppenhein et al. (J. Immunol., 116, 1466 (1976)) using thymus cells of a mouse of C3H/HeJ strain.

### (2) Determination of GIF activity

Portions (0.12 ml) of the test solution diluted to varying concentrations were placed into the wells of a 96-well microplate (Corning Co., Ltd), and 0.1 ml of Eagle's MEM suspension containing 10% FCS (fetal calf serum) containing human melonoma cells A375 in an amount of $2 \times 10^4$ cells/ml were then placed into each well, and the cells were incubated in a $CO_2$ incubator (Napco Co., Ltd.) for 4 days. After the incubation, 0.05 ml of 0.05% Neutral Red (Wako Junyaku Co., Ltd.) were placed into each well, followed by an incubation at 37°C for 2 hours. After removing the supernatant, 0.3 ml of phosphoric acid buffered saline were gently poured into each well for washing. After removing the wash solution, 0.1 ml of a mixture of monobasic sodium phosphate and ethanol in equal amounts were placed into each well, the plate was shaken for several minutes with a micromixer, and the amount of the dye absorbed by the cells was measured as an absorbance at 540 nm using a photometer for 96-well microtitration plates (Titer check multiscane, product of Flow Lab.) to determine the growth inhibition activity. The test group exhibiting 50% of the inhibition of cell growth of the control group, i.e., the test group which exhibited 1/2 the absorbance measured of the control group, was identified. The reciprocal of the number of times of dilution for the test group was taken as the GIF activity unit. Accordingly, when the GIF activity is 10 units, for example, the test solution, if diluted tenfold, still has the activity to inhibit the cell growth by 50%.

The following drawing is referred to in examples.

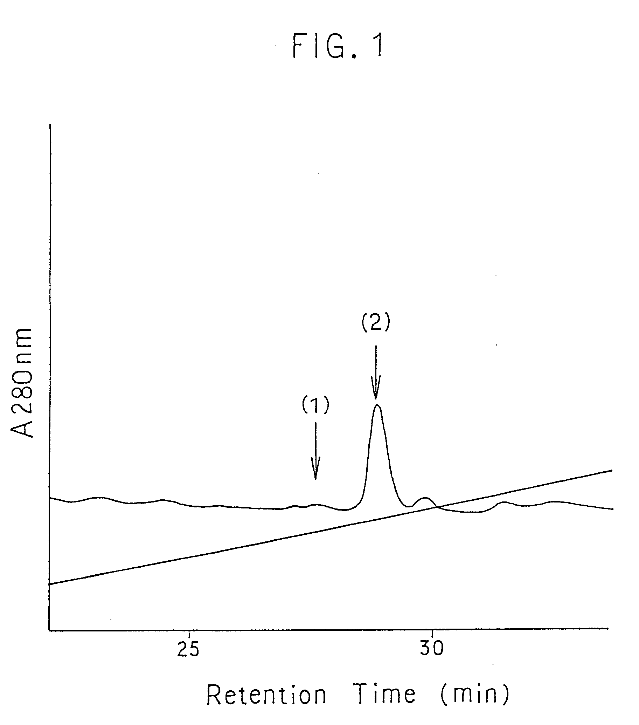

Fig. 1 is a chart showing the liquid chromatography analysis conducted using the desired polypeptide obtained according to Example 2.

Example 1

Vector ptrpGIF-α for cytoplasmic expression of human IL-1β (E. coli χ1776/ptrpGIF-α:FERM BP-949, deposited under the Budapest Treaty at Fermentation Research Institute (FRI), Agency of Industrial Science and Technology of 1-3, Higashi 1-chrome, Yatabe-machi, Tsukubagun Ibaraki-ken, 305, JAPAN on December 12, 1985 and disclosed in European Patent Application published under publication number 237967) was introduced into the E. coli strain HB101. The transformant thus obtained was also deposited under the Budapest Treaty at FRI on October 7, 1988 under the deposition number FERM BP-2089. LB medium (200 ml) of the following composition was inoculated with a loopful of such cells and shaken overnight at 37°C to incubate the cells and obtain a pre-incubated culture. Composition of LB medium

| Bacto-trypton (Difco) | 10 g/liter |
| Bacto-yeast extract (Difco) | 5 g/liter |
| NaCl (Wako Junyaku Co., Ltd.) | 10 g/liter |
| (pH) | (7.5) |

A production medium having the composition given below was inoculated with a specified amount of the above pre-incubated culture, which was then incubated in a jar fermenter under the conditions listed in Table 1 below at one of different rates of aeration. Composition of production medium

| $Na_2HPO_4 \cdot 12H_2O$ | 6 g/liter |
| $KH_2PO_4$ | 3 g/liter |
| NaCl | 0.5 g/liter |
| $NH_4Cl$ | 1 g/liter |
| Bacto-Casamino acid | 10 g/liter |
| Bacto-yeast extract | 0.5 g/liter |
| L-Cysteine·HCl | 75 mg/liter |
| L-Proline | 75 mg/liter |
| L-Leucine | 75 mg/liter |

The above medium was adjusted to a pH of 7.4 with 4N NaOH, and thereafter autoclaved at 121°C for 30 minutes or heated with steam at 123°C for 20 minutes for sterilization. Subsequently, the following components as sterilized separately were aseptically added to the medium at the time of the inoculation.

| 1M $MgSO_4 \cdot 7H_2O$ | 2 ml/liter |
| 1M $CaCl_2 \cdot 2H_2O$ | 0.1 ml/liter |
| 7.5 mg/ml Thiamine·HCl | 1 ml/liter |
| 40% Glucose | 18.75 ml/liter |

Table 1

| Incubator | Jar fermenter (Marubishi MSJ-U3) |
|---|---|
| Medium | Above production medium |
| Amount of medium | 20 liters |
| Incubation temp. | 37°C |
| Aeration | 0.25 vvm or 0.5 vvm |
| Stirring | 200 r.p.m. |
| pH | Initially 7.0 (after sterilization) |
| Amount of inoculant | Pre-incubated culture 400 ml/20 liters medium |
| Incubation time | 8 hours |

During the incubation, the culture medium was checked for the concentration of dissolved oxygen using a dissolved oxygen meter (Marubishi Bioengineering Co., Ltd.). When the aeration was conducted at 0.25 vvm, the concentration of the dissolved oxygen in the medium during the logarithmic growth phase was at least 0% saturation, i.e., in the range of about 5% to about 0% of the saturation concentration. When the aeration was conducted at 0.5 vvm, the concentration of dissolved oxygen medium during the logarithmic growth phase was at least 20% saturation and typically varied from about 25% to 20% of the saturation concentration.

After the completion of the production incubation, a 10 ml portion of the culture was configured at 10000 x g for 10 minutes to collect the cells, which were then suspended in 1 ml of 1M $Na_2HPO_4$ (product of Wako Junyaku Co., Ltd.) and allowed to stand at 4°C for several hours. The suspension was thereafter sonicated. The resulting cell fragments were centrifuged at 10000 x g for 10 minutes to collect the supernatant.

500 μl of 100 mM $CH_3COONa$ (pH 5.5) were added to 500 μl of the supernatant, the mixture was allowed to stand at 4°C for 30 minutes, and the resulting precipitate was filtered off with a 0.22-μm filter (product of Millipore). The filtrate was subjected to liquid chromatography (using Ultrochrom GTi (product of LKB); column TSK Gel SP-5PW, 7.5 x 75 mm; adsorption buffer 50 mM $CH_3COONa$ (pH 5.5); elution 50 mM $CH_3COONa$-0.5M NaCl (pH 5.5) gradient) to obtain fractions of Met(+) IL-1β and Met (-) IL-1β. The Met(+)/Met(-) ratio was calculated from the area ratio of a chart obtained. Table 2 shows the result.

Table 2

| Rate of aeration | Met(+)/Met(−) ratio |
|---|---|
| 0.25 vvm | 0.079 |
| 0.5 vvm | 0.385 |

Table 2 shows that the lower aeration rate results in an exceedingly smaller proportion of Met(+) produced, making it possible to selectively produce Met(-)

Example 2

E. coli HB10 strain (HB101/ptrpGIF-α-71S; FERM BP-1296; deposited under the Budapest Treaty at FRI on February 20, 1987 and disclosed in European Patent Application sublished under publication number 237967) transformed by vector ptrpGIF-α-71S for cytoplasmic expression of IL-1β derivative, i.e., human IL-1β wherein the 71st cysteine (Cys) was replaced by serine (Ser) was incubated in the same manner as in Example 1 using a Hitachi jar fermenter (200 liters) at an aeration rate of 0.5 vvm, followed by the same treatment and the same liquid chromatographic procedure as in Example 1. The dissolved oxygen

7

concentration in the medium during the logarithmic growth phase was about 5 to about 0% of the saturation concentration.

Fig. 1 shows the chromatographic chart obtained. In this chart, Met( + ) is represented by (1), and Met(-) by (2).

Fig. 1 reveals that the method of the invention selectively gives Met(-).


**Claims**

1. A method of incubating cells expressing a recombinant protein or polypeptide wherein the expressed protein or polypeptide is obtained without the N-terminal Met-residue encoded by the translation initiation codon which comprises incubating in a culture medium a host cell transformed with a cytoplasmic expression vector containing the gene of a protein or polypeptide, wherein the amount of oxygen dissolved in the culture medium is at most about 20% of the saturation concentration.

2. The method according to claim 1 wherein the polypeptide is a lymphokine.

3. The method according to claim 1 wherein the polypeptide is interleukin-1, interleukin-2, interleukin-3, interleukin-4, interleukin-5, interleukin-6, a colony stimulating factor, the tumor necrosis factor or a lymphotoxin.

4. The method according to any one of claims 1 to 3 wherein the host cell is E. coli, Bacillus subtilis, Diplococcus pneumoniae, yeast or actinomycetes.

5. The method according to any one of claims 1 to 4 wherein the host cell is E. coli.

6. The method according to any one of claims 1 to 5 wherein the polypeptide is interleukin-1 and the host cell is E. coli

7. The method according to claim 6 wherein the interleukin-1 is interleukin-1$\alpha$ or interleukin-1$\beta$.

8. The method according to any one of claims 1 to 7 wherein the gene is under the control of the $\lambda P_L$, $\lambda P_R$, trp, lacC, tufB, recA or 1pp promoter.

9. The method according to any one of claims 1 to 8 wherein the gene is under the control of the polypeptide trp promoter.

10. the method according to any one of claims 1 to 9 wherein the amount of oxygen dissolved in the culture medium is about 0 to about 5% of the saturation concentration.

11. The method according to any one of claims 1 to 10 wherein the amount of oxygen dissolved in the culture medium is controlled in the logarithmic growth phase of the culture.

FIG. 1